# EUROPEAN PATENT SPECIFICATION

(11) **EP 2 671 506 B1**
(45) Date of publication and mention of the grant of the patent: **05.04.2017**
(21) Application number: 12709173.4
(22) Date of filing: 06.02.2012
(51) Int. Cl.: A61B 5/0408, A61B 5/0478, D02G 3/44, D04B 1/00

(54) **ELECTRODES BASED ON TEXTILE SUBSTRATES**
ELECTRODES BASED ON TEXTILE SUBSTRATES
ÉLECTRODES À BASE DE SUBSTRATS TEXTILES

(30) Priority: 04.02.2011 PT 2011105517
(43) Date of publication of application: 11.12.2013
(73) Proprietor: Universidade do Minho, 4704-553 Braga (PT)
(72) Inventor: DE ALMEIDA WHITEMAN CATARINO, André Paulo, 4800-058 Guimarães (PT); DE JESUS DIAS, Maria, 4800-058 Guimarães (PT); TEIXEIRA CARVALHO, Helder Manuel, 4800-058 Guimarães (PT); MOREIRA FERREIRA ROCHA, Ana Maria, 4800-058 Guimarães (PT)
(74) Representative: Ferreira, Maria Silvina
(86) International application number: PCT/IB2012/050537
(87) International publication number: WO 2012/104826

(56) References cited:
- WO-A2-01/02052
- GB-A- 2 462 205
- US-A- 4 983 814
- US-A1- 2010 234 715
- SILVA M ET AL: "Study of vital sign monitoring with textile sensors in swimming pool environment", IECON 2009 - 35TH ANNUAL CONFERENCE OF IEEE INDUSTRIAL ELECTRONICS (IECON 2009) - 3-5 NOV. 2009 - PORTO, PORTUGAL, IEEE, PISCATAWAY, NJ, USA, 3 November 2009 (2009-11-03), pages 4426-4431, XP031629552, ISBN: 978-1-4244-4648-3
- M SILVA ET AL: "Textile SENSORS FOR ECG and RESPIRATORY FREQUENCY ON SWIMSUITS", IMTC 2009, ESITH, CASABLANCA MOROCCO, 14 November 2009 (2009-11-14), pages 301-310, XP55024910,
- MARIA JOSÉ ABREU ET AL: "Integration and embedding of vital signs sensors and other devices into textiles", INTERNATIONAL CONFERENCE AND EXHIBITION ON HEALTHCARE & MEDICAL TEXTILES (THE MEDTEX07), BOLTON, 4, 2007 - "INTERNATIONAL CONFERENCE AND EXHIBITION ON HEALTHCARE & MEDICAL TEXTILES", 31 July 2007 (2007-07-31), XP55024922,

## Description

### Field of the invention

Present invention relates to textile electrodes for vital signals monitoring. More specifically it relates to a fabrication process of textile electrodes based on weft knitting, their integration into clothing and application as sensor for measuring the electrical potential of an individual, such as ECG, EMG, EEG and respective transmission to a monitoring system.

### Background of the invention

To measure electrical potentials such as ECG-electrocardiography, EMG - electromyography, EEG electroencephalography, electrodes are used in contact with the individual skin to detect the electrical signal. This signal is transmitted to an electronic system which keeps and/or visualizes the information in a rough or detailed form.

In the past few years two monitoring applications were identified: medical applications, which require a high quality level of the signal detected by the electrodes and well-being applications. In this latter case, the signals may not need the same high quality level of those required for the medical application.

Signals' monitoring systems can be divided into portable and non-portable. These latter are specially used in the clinical activity but the first ones can be used either for medical or well-being purposes to constantly monitor an individual.

From the electrodes point of view it is conventionally accepted that they should be composed by silver/silver chloride (Ag/AgCl) because globally it promotes a better signal quality. Thus electrodes are pieces with a skin contact surface made of a homogeneous, rigid or flexible material.

The main advantages of flexible electrodes are their better accommodation to the skin and free positioning due to the fact that they are solely linked to the measuring system. To keep these electrodes on position, adhesives are used and to improve conductivity, i.e., to reduce skin-electrode impedance, it is common to use electro conductive gels. Skin irritation caused by the adhesives after prolonged use is their major disadvantage.

To avoid the use of adhesives and make these monitoring systems more user-friendly, integration of electrodes onto or into clothing was tried.

Several solutions of textile electrodes, namely based on knitted and woven substrates were proposed. Conductive yarns interlaced or interwoven into structures produce electrodes that when connected to measuring systems allow registering electrical potentials. However, it happens that the electrical properties of textile structures vary with strain that degrade the quality of the electrical signal. It has also been verified that to keep the contact between the electrode and the skin without adhesives, the clothing piece should promote compression to guarantee the correct positioning of the electrode and assist the permanent contact with the skin. To assure the contact and, consequently, the quality of the signal, the existing solutions use foams, silicones or other products that force the contact of the electrode with the skin.

The interconnection/communication interface between the electrode and the measuring systems is equally fundamental to assure the quality of the signal. Several approaches are described in the state of the art, the most popular using press studs, seams or glue. Data lines may be regular insulated electric cables attached to the clothing piece, conductive yarns sewed onto the pieces or conductive yarns integrated into the piece itself. The sensors can be attached to the clothing pieces through sewing or embroidery or produced into the piece itself during production, in specific regions meant to be electrodes.

In what concerns electrodes' base-structures, the existing know-how on their influence on the quality of the signal is still incipient.

The majority of the existing textile electrodes use simple structures, such as plain weave and rib knit and aim essentially at ECG measurement.

The patent documents US7474910, US7308294 and
US20070127187 describe a type of electrodes based on textiles with conductive and non-conductive yarns with associated elasticity. The electrode described is composed of two fabric plies, both with conductive regions, superimposed so that the signal measured by the internal conductive ply is transmitted to the external one, also conductive. This latter fabric ply is connected through press studs to the electronic system that registers the bioelectric potentials.

Both inventions were designed to ECG signals measurement. The description of the structure is vague, but the studies performed indicate that there is an interaction between raw material-structure that can influence the quality of the signal.

The patent document US20060183990 describes a system to measure the signals coming from bioelectric potentials through electrodes attached to a piece of clothing after production. No mention to the used structures or fabrication process. In this invention special emphasis to the central part of the clothing piece, a T-shirt, is made which is produced to apply an adequate compression to guarantee the contact between the electrodes used to measure potentials and the skin. The invention doesn't describe the electrodes production method stating solely that they are textile electrodes.

The patent document US6970731 describes sensors to monitor vital signals. These sensors produced on textiles, woven or knitted, can be used standalone or integrated in clothing. The sensor is composed by a conductive gel, a textile substrate and a connector or press stud to which an electronic system is connected. Two basic structures are described, one in woven fabric-plain weave and another in weft knitted fabric- 1x1 rib. Particular relevance is made to the contact skin-electrode without adhesives. It is visible from the text that the electrodes are not integrated into de clothing piece.

The patent document US20050034485 describes a piece of clothing to measure biological parameters which is based on the attachment of sensors made of conductive yarns to measure ECG and on tracks produced by elastic conductive yarns that transport the signals with data and energy to these sensors. The fabrication process is not mentioned and the textile structure used not described.

The patent document WO 2007/050650 describes the weaving and knitting processes of fabrics able to act as a support infrastructure to sensors used to measure the vital parameters of an individual. The positioning of the sensors produced with conductive yarns and using a flat weft knitting machine SHIMA SEIKI, as well as, the integration of connecting tracks into the piece of clothing for data transmission are described.

The patent document WO 2009/013704 describes a textile-based electrode that combines more than one textile technology to be produced. The electrode is composed by two superimposed layers, one woven fabric and the other, which is in contact with the skin, a knitted fabric. To promote the contact with the skin a gel support is placed next to these two conductive layers and covered by a third layer, sewed or linked by another method to the base substrate of the electrode.

The patent document US 2007/0083096 describes a piece of clothing to monitor several biological parameters, among them ECG. To do so, well determined regions are built in the piece of clothing using conductive and elastic yarns to produce electrodes. The electrodes are fabricated on a rib knitting machine. The structure used in the electrodes is not exactly defined.

The search that was carried out and the preceding documents demonstrated the limitations and the problems related to the construction of electrodes based on textile substrates. It is the intention of the present invention to solve those issues: permanent contact with the skin; possibility to utilize as dry electrode; interface/interconnection with the measuring system; adequate structure and complete integration into the piece of clothing.

The developed method, using specific knitted fabric structures, makes the most of weft knitted fabric flexibility and conformability towards body skin, as well, as tri-dimensionality to impart thickness and guarantee the permanent contact with the skin.

Electrode's construction also assures an adequate insulation and an easy access to connect the electronic components. The integration into pieces of clothing is direct due to the textile technology used, specially designed to underwear. The production process of the structure for ECG and EMG measuring electrodes is completely characterized to guarantee a quality signal.

### General description of the invention

Present invention concerns the development of a textile-based electrode which allows the measurement of electrical potentials and assures the compression needed to maintain its contact with the skin to obtain a good quality signal.

The record of biological signals such as electrocardiography - ECG, electromyography - EMG and electroencephalography - EEG are the prospective applications.

This invention can be used as a standalone device or integrated into the piece of clothing. In this latter case, the piece of clothing carries the electrical potentials' measuring system.

This invention is characterized by a tri-dimensional textile electrode which results from a tubular structure where a pre-defined area composed by conductive yarns is formed. The electrode obtained by this method is smooth to the skin and ensures a permanent and effective contact with it. Moreover, the insulating layer around the electrode avoids artifacts capture and contributes to keep a moistened microclimate in the sensor area which improves the quality of the signal.

The electrode structure is produced on a weft knitting machine with one or two needle systems. The fabrication process uses conductive yarns, non-conductive ground yarns and elastomeric yarns, such as elastane. The process combines yarns' feeding and withdrawal, according to a predetermined sequence, with knitted fabric structure, as represented by the pattern repeat or stitch structural unit.

The fabrication sequence allows the formation of a knitted tube effect responsible for projecting the electrode to the outer face of the fabric when tensioned both vertically and horizontally. This imparts tri-dimensionality to the structure. The use of elastomeric yarns promotes the required compression effect to better adjust the electrode to the skin and assure the correct position.

The electrode is produced using electrically conductive yarns based on raw materials, such as steel, silver or conductive polymers, together with elastomeric yarns, such as elastane.

When steel is used, this is blended, in a certain percentage, to another non-conductive fibre, such as polyester. Bekintex from Beckaert is an example of this type of yarn.

When silver is selected, generally a multifilament polymeric yarn, such as polyamide, is used and each filament is coated by a thin film of silver. The resulting yarn may cover or not an elastomeric yarn. If this silver-coated yarn does not have an elastic core, it should have the lowest possible crimp and be flat to obtain a more stable electrode.

Elitex yarns are examples of silver-coated yarns with an elastic core. The elastomeric yarn in the core will contribute to oppose to knitted fabric extensibility and to reinforce the projection effect to the third dimension.

The main limitations to the production of textile electrodes are the mechanical characteristics of the conductive yarn and respective raw material.

Thus, the yarn should have low flexural rigidity and adequate tensile strength to be able to bend and support the traction forces imposed by the knitting machine and during use. It should demonstrate the required elasticity to be able to recover its original shape after force removal. Yarn diameter should be compatible with the needles size used on knitted fabric production. Examples of yarns with these characteristics are: synthetic multifilament and steel fibre yarns, conductive core yarns, synthetic yarns doped with conductive substances (such as carbon) or silver-coated synthetic filament yarns.

Two types of yarns were selected to produce the electrodes:
One based on a blend of polyester (variable, specifically 80%) and steel (variable, 10-30%, specifically, 15-25%, specifically 20%) and another based on a silver-coated polyamide with an elastane monofilament core.

From these experiments the first versions of ECG and EMG electrodes resulted.

Elasticity, particularly in the transverse direction, is one of the most relevant characteristics of knitted fabrics.

The electrode, as an integral part of the knitted fabric, will be extended by tensile forces. These extensions can cause the following effects: increase the measuring area and change the distance between the electrodes; significantly vary electrode's conductive properties and take too long to recover its original dimension after force release. These phenomena need to be very well characterized.

Keeping the contact between the electrode and the skin is a requirement to guarantee the quality of the signal: in surface EMG it is usual to clean the skin and apply a conductive gel to reduce the electrode-skin impedance.

To avoid electrode displacement it is common to use adhesives.

In the present invention maintaining the contact between the electrode and skin and its correct positioning are guaranteed through the compression promoted by the structure tri-dimensionality and through the combination of yarns that constitute the electrode. No adhesives are used.

In situations where the risk of the electrode to lose contact with the skin is higher, as in intense physical activities, contact robustness can be increased adding a support element, preferably foam or a flexible silicone. This element inserted after production into the tube formed by the electrode will reinforce the contact with the skin.

Electrode's insulation is achieved through coating with an insulating product, such as an elastic silicone.

The insulator is applied in the outer face of the knitted fabric that incorporates the electrode and in the inner face next to electrode's boundaries, specifically in the interface region.

Adding this insulator also promotes moisture retention within the area of the electrode, which leads to a better quality of the signal.

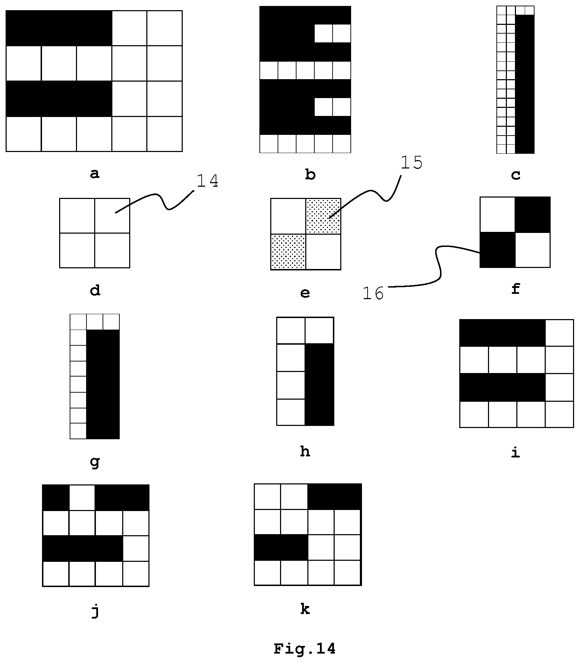

The quality of the signal obtained with the knitted structures illustrated in figure 14 (a) till (k) is very close to the one obtained with conventional electrodes.

The proposed electrode can be connected to regular electrical cabling or to tracks knitted with conductive yarns in the fabric itself for signal transmission to an electronic system.

The sequence of tracks construction makes easy access to the conductive yarns which can be connected to the electrode by attachment, gluing or by another joining method.

As an example, two textile electrodes (referred to as open and close versions) are presented.

In what concerns skin-electrode impedance, as well as, signal acquired with tensioned and relaxed muscle, the results are similar to those obtained with the commercially available flexible or rigid electrodes.

The electrodes produced by this method can be used in any application where bioelectrical signals are to be measured, such as electromyography (EMG), electrocardiography (ECG), among others.

### Description of the figures

Figure 1 - Perspective of the electrode (8) closed version in tube form with a central axis AA' and respective connecting points (9) between courses B and C; where (2)corresponds to the conductive region ; (3) to the support for the inner part of the tube formed by the conductive region (2) and the non-conductive region (4).
Figure 2 - Technical face of the electrode (8), in open version, where (2) corresponds to the conductive region ;(4) the non-conductive region; (5) the interface between conductive and non-conductive regions and (8) the electrode.
Figure 3 - Technical back of the electrode (8), in open version, where the conductive yarns (6) are detailed to show the accessibility to data lines connection, where (2) corresponds to the conductive region and (4) to the non-conductive region;
Figure 4 - Side view of electrode's section (8) in open version, with the insulating layer (1) incorporated in the technical face and back; (2) the conductive region; (4) the non-conductive region and (5) the interface between conductive and non-conductive regions;
Figure 5 - Side view of electrode's section (8) in open version, with the conductive yarns accessible (6) and the insulating layer (1) incorporated in the technical face and back; where (2) corresponds to the conductive region; (4) to the non-conductive region and (5) the interface between conductive and non-conductive regions.
Figure 6 - Technical face of the electrode (8) in open version, detailing the connection between courses B and C where (2) corresponds to the conductive region; (4) to the non-conductive region and (5) the interface between conductive and non-conductive regions.
Figure 7 - Side view of electrode's section (8) closed version in tube form integrating the conductive region (2) and the non-conductive region (4).
Figure 8 - Side view of electrode's section (8) closed version in tube form with the inner support element (3), the conductive region (2) and the non-conductive region (4).
Figure 9 - Side view of electrode's section (8) closed version in tube form, where (3) is the inner support element; (1) the insulating material incorporated in the technical face of the knitted fabric, (2) the conductive region and (4) the non-conductive region.
Figure 10 - Side view of electrode's section (8) closed version in tube form, where (3) is the inner support element, (1) the insulating material incorporated in the technical face and back of the knitted fabric, (2) the conductive region and (4) the non-conductive region.
Figure 11 - Illustration of the application of the electrode (8) in open version to measure ECG where (9) corresponds to the tie-in loops to connect courses B and C; (10)the track- channels for the electrical signals conductors; (11) the electric cables or conductive yarns and (12) the base- clothing piece, non-conductive and with elastic properties.
Figure 12 - Illustration of the application of the electrode (8) in open version to measure surface EMG, for example in the leg or thigh, where (12) is the base-clothing piece (13) the data lines comprised by insulated conductors or tracks knitted with conductive yarns and electrically insulated.
Figure 13 - Detail of electrodes (8) used to measure surface
   EMG in thigh muscles, where (12) is the base- clothing piece (13) the data lines comprised by insulated conductors or tracks knitted with conductive yarns and electrically insulated.
Figure 14 - Pattern repeat of the knitted fabric structures used in the production of the electrodes (8), where the horizontal lines represent the courses, the vertical lines the wales, (14) represents the plain loop, (15) the tuck loop and (16) the miss loop (float).

### Detailed description of the invention

The present invention uses seamless circular knitting machines with a needle system. This knitting machine allows the production of the electrode with the intended shape and size, in the required position in the clothing piece and with unique stability and elasticity properties. With this technology electrodes for electrocardiography, electromyography or other electrical potential measurement can be developed, only by defining adequate dimensions, shape and positioning.

In the case of measuring muscular activity through surface EMG, electrodes must be aligned with the muscle fibres' bundles. This technology also enables the production of electrodes with the required alignment.

As electrodes are embedded in the knitted fabrics user comfort is guaranteed. Electrodes are reusable and can be cleaned/washed without losing their functions.

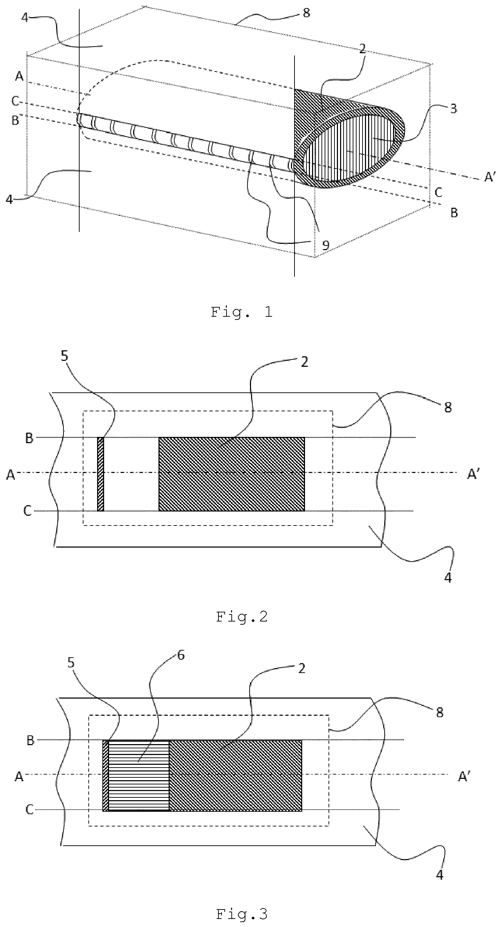

Figure 1 illustrates the electrode in close (tube) version. The conductive region (2) composed by conductive yarn and elastane; the optional support element (3) which increases the contact ability between the electrode and the skin; and (4) the base knitted fabric of non-conductive material where the electrode is embedded. Figures 2 and 3 illustrate how the electrode is knitted. To produce the electrode, the conductive yarn in (4) is substituted by the conductive yarn in (2) and (5). The operation sequences that will be presented are applicable to any structure pattern repeat, such as the ones showed on figure 14: In the structures' courses, where the electrode is going to be produced,
a) the conductive yarn is lowered to the knitting position, to join to the non-conductive yarn that is being knitted to produce the region (5);
b) the conductive yarn leaves the knitting zone to produce a yarn float, which will be the connecting yarn(6);
c) the conductive yarn is lowered again to the knitting position, to join to the non-conductive yarn that is being knitted;
d) the non-conductive yarn leaves the knitting zone after 5 to 10 needles to ensure a connection between the ground yarn a and the conductive yarn;
e) the conductive region (2) of the electrode (8) is produced with the structure defined by one of the pattern repeats illustrated in figure 14;
f) after the production of the conductive region (2) of the electrode (8), the non-conductive yarn re-enters the knitting zone, to join to the conductive yarn;
g) the conductive yarn leaves the knitting zone after 5 to 10 needles to ensure the connection to the non-conductive yarn;
h) the dial knifes cut the yarn in excess. The non-conductive region (4) in the opposite side of the electrode is thus formed;
i) the elastane yarn never leaves the knitting zone. The in and out of the conductive yarn at the beginning of the knitting process allows releasing conductive yarn for connection (6) to regular conductors, insulated or not, using any joining method. These conductors transmit the electrical signal to the signal conditioning system.

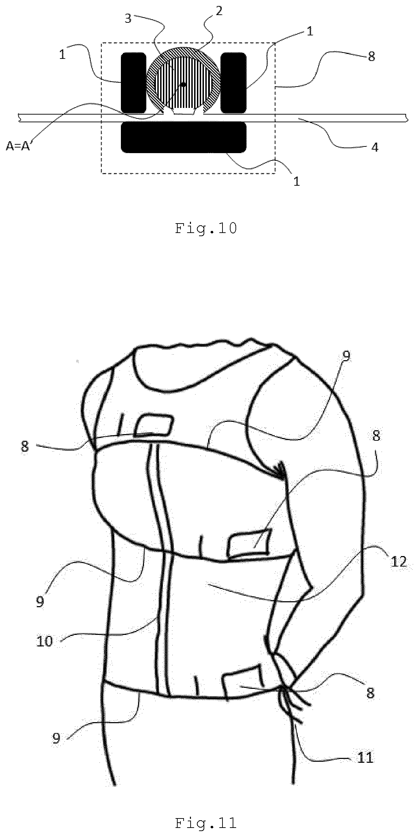

In figures 11, 12 and 13 the preferred joining method was by mechanical attachment and subsequent insulation with silicone or Teflon. The conductor used to transmit the signal detected by the electrode is copper multifilament covered by a silicone layer.

Other joining methods are possible such as: (i) press stud; (ii) conductive glue; (iii) insulating glue or (iv) a knitted area with conductive yarn. In this latter case (iv), immediately before electrode's production, a locknit structure with conductive yarn is knitted with an area that will be used to insert for example a conductive press stud. The conductive yarn is then connected to the electrode through this press stud. Another method to connect the electrodes to the electronic conditioning system is based on knitting several courses with conductive yarn-conductive tracks until electrodes' region is reached. This track works as a conductor and can be electrically insulated from the external environment or not.

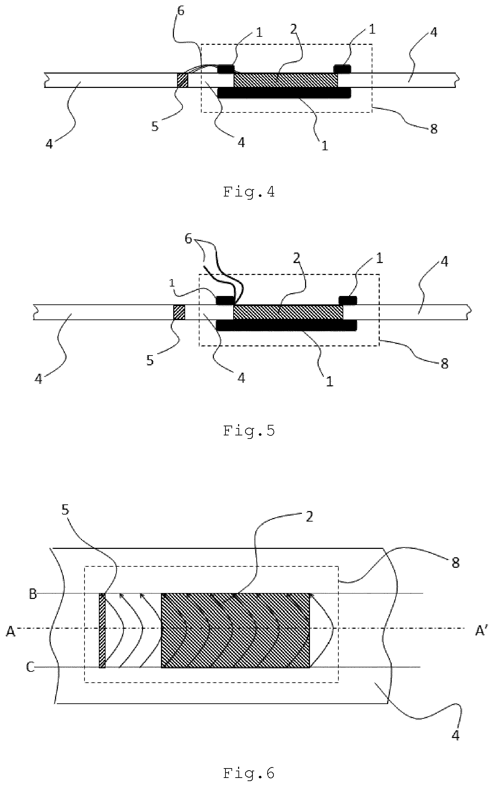

Figure 4 shows the section view along the AA' axis of the electrode illustrated in figures 2 and 3.

Two non-conductive regions are identified (4), before and after the first region with conductive yarns (5), connecting conductive yarns (6) and the electrode (8). The silicone insulating layer (1) can be applied in the technical face - bigger area under the conductive region (2) of the electrode (8) or in the technical back - smaller area lateral to the conductive region (2) of the electrode(8).

The conductive yarn for connection (6) is cut after production and is identified in figure 5.

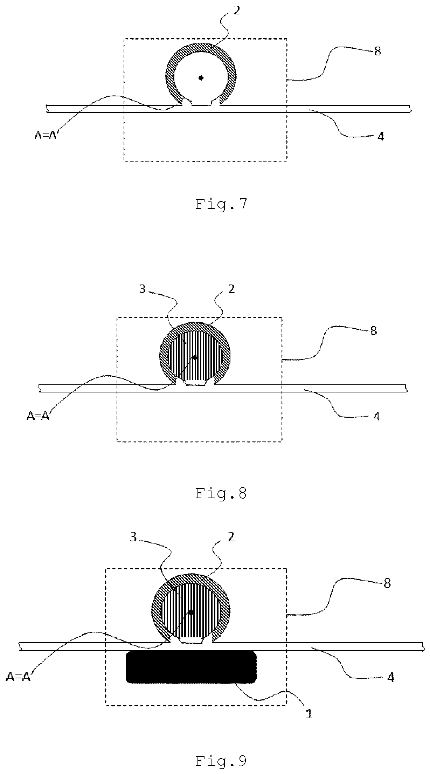

The pattern repeat can be knitted in the horizontal or vertical directions within the conductive region (2) of the electrode (8) as many times as needed to produce the electrode with the size and shape required. To obtain the electrode illustrated in figure 1, projected to the third dimension, courses B and C identified in figures 2 and 3 must be connected. To do this and following the previous described phases, also illustrated in figures 2 and 3, an additional phase illustrated in figures 6 and 7 is required. These figures show that the connection between courses B and C allow the production of a cylindrical tube whose axis is AA'. This connecting operation is performed with a specific structure in which several needles keep the loops and don't knit while regions (2), (4) and (5) are produced. At the end all needles knit producing the tube represented in figures 1 and 7.

Figure 1 shows the connecting points (9) between courses B and C, as well as the electrode's conductive region (2) and the two non-conductive regions (4) at its boundaries. Figures 7, 8, 9 and 10 show the different alternatives to the construction illustrated in figures 6 and 7.

Figure 8 illustrates the cross section of the electrode's tube and the two non-conductive regions (4) at its borders. Adding a support element (3) to increase electrode's thickness can be observed in figure 9. The support element is inserted after production through the tube opening. An insulating layer (1) can also be applied to the electrode in the technical face as illustrated in figure 9, or in the technical back as shown in figure 10. The complete version and the optimised tri-dimensional shape of the electrode is obtained in this way.

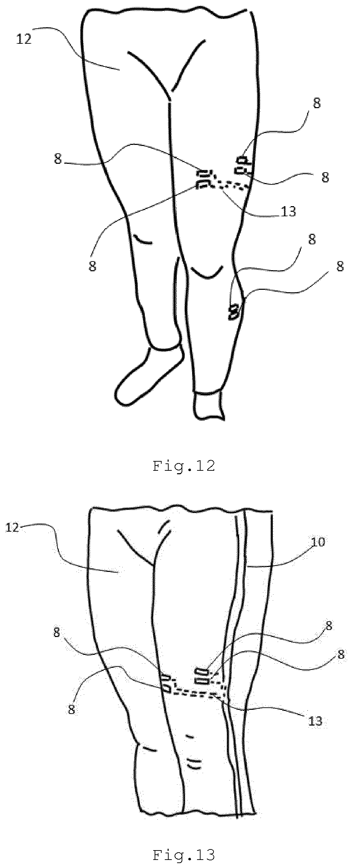

Figure 11 illustrates an application of the electrode to measure ECG signals. Electrodes are shown from figures 2 to 5. The joining to the measuring system was made by mechanical attachment of the connecting yarns (6) to the electrical conductors, later insulated with silicone. The yarns carrying the ECG signal are inserted in track-channels (10) specially designed in the clothing piece. The clothing piece and the electrodes are simultaneously produced. Figures 12 and 13 illustrate EMG electrodes of the same type of the ECG ones, but with the dimensions adequate to EMG measurement according to SENIAM.

Electrodes are also connected to data cables inserted in track-channels (13) and (10) specially designed in the piece of clothing. The clothing piece and the electrodes are simultaneously produced.

As previously mentioned electrodes are produced on a seamless knitting machine. Yarn feeding should be made by an intermittent yarn feeder. Yarn tensions in the electrode's area will depend on the characteristics of the conductive yarn, namely yarn linear density. Preferably a yarn input tension between 0,1 -0,15 cN/tex should be used, with a minimum of 2 cN. Elastane feeding tension equally depends on its linear density, varying preferably between 2 and 4 cN. An electronic yarn feeder is preferred for this type of filament to be able to adjust in real time the elastane input tension. The ground yarn, around the electrode, can be of any raw material fed by an intermittent feeder and the yarn input tension adjusted as previously mentioned. In this case it was 2 cN as yarn linear density was lower than 20tex.

The described method allows also the production of arrays of electrodes, used for example in EMG measurement. The production process is similar to the one described. The difference relies on the insertion of ground yarn courses between the electrodes. The number of inserted courses to intercalate the electrodes will depend on the yarn characteristics, the knitting machine, user or official institution specifications, such as SENIAM. On the present case the electrodes' array for EMG comprises eight electrodes, spaced two by two 1 cm, as specified by the above-mentioned official institution. These electrodes were connected to the developed signal conditioning system through copper conducting lines attached to them.

## Claims

1. Électrode textile (8) configurée afin d'incorporer une couche interne en tissu maillé en trame (4) afin de mesurer les potentialités électriques personnelles d'un usager, sachant que l'électrode textile (8) comprend :
- Un élément de support tubulaire (3) comprenant un axe longitudinal AA') ;
- Une région conductrice tubulaire (2), composée d'un fil conducteur et d'élasthanne et entourant radialement l'élément de support tubulaire (3);
- Deux régions non-conductrices (4), délimitant la dite région conductrice (2) en direction longitudinal axialement ;
- Une couche isolante en silicone (1), appliquée sur les bords latéraux ou derrière la région conductrice tubulaire (2).

2. Électrode, selon la revendication 1, où les fils conducteurs comprennent de l'acier ou de l'argent.

3. Électrode, selon la revendication 2, où les fils conducteurs en acier comprennent un fil combiné en fibre non-conductrice, mélangé avec 10 - 30% de fibre d'acier.

4. Électrode, selon la revendication précédente, où le pourcentage de fibre d'acier du fil mélangé est compris entre 15 - 25%.

5. Électrode, selon la revendication précédente, où le pourcentage de fibre d'acier dans fil mélangé visé est de près de 20%.

6. Électrode, selon la revendication 2, où les fils conducteurs en argent comprennent des filaments de polymère revêtus d'argent.

7. Électrode, selon la revendication précédente, où le polymère susmentionné est en polyamide.

8. Électrode, selon la revendication précédente, où l'élément de support (3) est en mousse ou en silicone.

9. Méthode de production de l'électrode textile selon une quelconque revendication 1 à 8, comprenant un tissu maillé en trame conducteur et tubulaire (2) délimité par deux régions non-conductrices (4) et une couche isolante en silicone (1), appliquée sur les bords latéraux ou derrière la région conductrice tubulaire (2), comprenant les étapes suivantes:
- Placer le fil conducteur dans la zone maillée, afin qu'il soit relié au fil non-conducteur qui est en train d'être maillé;
- Retirer le fil conducteur de la zone maillée pour produire un flottement avec le fil, afin que le fil conducteur soit disponible (6);
- Replacer le fil conducteur dans la zone maillée, afin qu'il soit lié au fil non-conducteur qui alimente les aiguilles;
- Retirer le fil non-conducteur de la zone maillée, après quelques aiguilles, afin que la connexion entre les fils conducteurs et non-conducteurs soit assurée;
- Créer la région conductrice (2) de l'électrode (8), en se fondant sur le report de motif de l'une ou de plusieurs boucles (flottées) manquées (16), boucles repliées (15) boucles unies (14), ou des combinaisons de celles-ci;
- Replacer le fil non-conducteur dans la zone maillée afin qu'il soit lié au fil conducteur;
- Retirer le fil conducteur de plusieurs aiguilles de la zone maillée après avoir garanti la connexion entre les fils conducteur et non-conducteur;
- Couper l'excès de fil, obtenant ainsi une région non-conductrice (4), à l'opposé de l'électrode (8); le fil élastomère reste dans la zone maillée comme indiqué dans les étapes antérieures;
- Appliquer une couche isolatrice comme du silicone en haut de la région conductrice (2) en face de l'électrode maillée et autour de la partie conductrice de l'électrode maillée sur sa partie arrière.

10. Méthode, selon la revendication 9, où les unités répétées de la région conductrice (2) de l'électrode (8) comprennent des structures maillées en trame dans lesquelles les lignes représentent le nombre de courses, les colonnes, le nombre d'aiguilles, un carré noir, une boucle (flottée) manquée (16), le carré noir et blanc, une boucle repliée (15) et le blanc, une boucle unie (14).

11. Méthode, selon les revendications 9 ou 10, où le nombre d'aiguilles lors du maillage simultané est compris entre 5 à 10 afin de garantir la connexion entre les fils conducteur et non-conducteur.

## Patentansprüche

1. Eine Textilelektrode (8), konfiguriert zur Einbettung in eine Gestrick-Grundschicht(4)zur Messung personenbezogener elektrischer Potenziale eines Anwenders, die Textilelektrode (8) umfassend:
- ein rohrförmiges Trägerelement(3) umfassend einer Längsachse (AA');
- ein rohrförmiger leitfähiger Bereich (2), bestehend aus leitfähigem Garn und Elastan und radial das rohrförmige Stützelement umgebend (3);
- Zwei nicht leitfähige Bereiche (4), welche den genannten leitfähigen Bereich (2) in einer axialen Längsrichtung abgrenzen;
- Eine Silikon-Isolierschicht (1), welche an den Seiten oder auf der Hinterseite des rohrförmigen leitfähigen Bereich (2) appliziert ist.

2. Elektrode, gemäß Anspruch 1, wobei die leitfähigen Garne Stahl oder Silber enthalten.

3. Elektrode gemäß Anspruch 2, wobei die leitfähigen Garne auf der Basis von Stahl eine nicht leitfähige Faser und ein Mischgarn, gemischt mit 10-30% Stahlfaser, umfassen.

4. Elektrode, gemäß dem vorangegangenen Anspruch, wobei der prozentuale Anteil von Stahlfaser in dem Mischgarn zwischen 15-25 % liegt.

5. Elektrode, gemäß dem vorangegangenen Anspruch, wobei der prozentuale Anteil von Stahlfaser in dem gennanten Mischgarn etwa 20 % beträgt.

6. Elektrode, gemäß Anspruch 2, wobei die auf Silber basierten leitfähigen Garne mit Silber beschichtete Polymerfilamente umfassen.

7. Elektrode, gemäß dem vorangegangenen Anspruch, wobei das genannte Polymer Polyamid ist.

8. Elektrode gemäß dem vorangegangenen Anspruch, wobei das Trägerelement (3) Schaumstoff oder Silikon ist.

9. Produktionsmethode der Textilelektrode gemäß der Ansprüche 1-8,
enthaltend ein rohrförmigesleitfähiges Gestrick mit einem leitfähigen Bereich (2), abgegrenzt durch zwei nicht leitfähige Bereiche (4) und eine Isolierschicht aus Silikon (1), appliziert an den Seiten oder auf der Hinterseite des rohrförmigen leitfähigen Bereichs (2), umfassend die folgenden Schritte:
- Positionierung des leitfähigen Garns im Strickbereich, welcher mit dem nicht leitfähigen Garn, welches gestrickt wird, zu verbinden ist;
- Entnahme des leitfähigen Garns aus der Strickzone zur Bildung eines Puffers mit dem Garn, sodass verbindendes Garn verfügbar ist (6);
- Neupositionierung des leitfähigen Garns im Strickbereich, welcher mit dem nicht leitfähigen Garn, welches die Nadeln bestückt, zu verbinden ist;
- Entnahme des nicht leitfähigen Garns aus der Strickzone, einige Nadeln später, sodass die Verbindung zwischen den nicht leitfähigen und leitfähigen Garnen gesichert ist;
- Herstellung des leitfähigen Bereichs (2) der Elektrode (8), welche auf dem Rapportmuster von einer oder mehr Miss (flottliegende) - Fäden, (16), Abnäher- Fäden (15), flachen Fäden (14) oder Kombinationen davon basiert;
- Neupositionierung des nicht leitfähigen Garns im Strickbereich, welcher mit dem nicht leitfähigen Garn zu verbinden ist;
- Entnahme des leitfähigen Garns aus der Strickzone einige Nadeln weiter, um die Verbindung zwischen den nicht leitfähigen und leitfähigen Garnen zu gewährleisten;
- Abschneiden des überschüssigen Garns, dadurch Zustandekommen des nicht leitfähigen Bereichs (4) gegenüber der Elektrode (8); das elastomerische Garn bleibt im Verlauf der vorangegangenen Schritte in der Strickzone;
- Auftragen einer Isolierschicht wie Silikon auf der Oberseite des leitfähigen Bereichs (2) im Bereich der Vorderseite der gestrickten Elektrode und Umschließen des leitfähigen Teils der gestrickten Elektrode auf deren Hinterseite.

10. Methode, gemäß Anspruch 9, wobei die Wiederholungseinheiten des leitfähigen Bereichs (2) der Elektrode (8) Gestrick-Strukturen umfassen, bei denen die Linien die Anzahl der Bahnen repräsentieren, die Reihen mit der Nadelanzahl, ein schwarzes Quadrat der Miss (flottliegende) Fäden, (16), das schwarze und weiße Viereck der Abnäher-Fäden (15) und die weiße flache Fäden (14).

11. Methode gemäß der Ansprüche 9 oder 10, wobei die Nadelanzahl des simultanen Strickens zwischen 5 bis 10 liegt, um die Verbindung zwischen nicht leitfähigen und leitfähigen Garnen zu gewährleisten.

## Revendications

1. Électrode textile (8) configurée afin d'incorporer une couche interne en tissu maillé en trame (4) afin de mesurer les potentialités électriques personnelles d'un usager, sachant que l'électrode textile (8) comprend :
- Un élément de support tubulaire (3) comprenant un axe longitudinal AA') ;
- Une région conductrice tubulaire (2), composée d'un fil conducteur et d'élasthanne et entourant radialement l'élément de support tubulaire (3);
- Deux régions non-conductrices (4), délimitant la dite région conductrice (2) en direction longitudinal axialement ;
- Une couche isolante en silicone (1), appliquée sur les bords latéraux ou derrière la région conductrice tubulaire (2).

2. Électrode, selon la revendication 1, où les fils conducteurs comprennent de l'acier ou de l'argent.

3. Électrode, selon la revendication 2, où les fils conducteurs en acier comprennent un fil combiné en fibre non-conductrice, mélangé avec 10 - 30% de fibre d'acier.

4. Électrode, selon la revendication précédente, où le pourcentage de fibre d'acier du fil mélangé est compris entre 15 - 25%.

5. Électrode, selon la revendication précédente, où le pourcentage de fibre d'acier dans fil mélangé visé est de près de 20%.

6. Électrode, selon la revendication 2, où les fils conducteurs en argent comprennent des filaments de polymère revêtus d'argent.

7. Électrode, selon la revendication précédente, où le polymère susmentionné est en polyamide.

8. Électrode, selon la revendication précédente, où l'élément de support (3) est en mousse ou en silicone.

9. Méthode de production de l'électrode textile selon une quelconque revendication 1 à 8, comprenant un tissu maillé en trame conducteur et tubulaire (2) délimité par deux régions non-conductrices (4) et une couche isolante en silicone (1), appliquée sur les bords latéraux ou derrière la région conductrice tubulaire (2), comprenant les étapes suivantes:
- Placer le fil conducteur dans la zone maillée, afin qu'il soit relié au fil non-conducteur qui est en train d'être maillé;
- Retirer le fil conducteur de la zone maillée pour produire un flottement avec le fil, afin que le fil conducteur soit disponible (6);
- Replacer le fil conducteur dans la zone maillée, afin qu'il soit lié au fil non-conducteur qui alimente les aiguilles;
- Retirer le fil non-conducteur de la zone maillée, après quelques aiguilles, afin que la connexion entre les fils conducteurs et non-conducteurs soit assurée;
- Créer la région conductrice (2) de l'électrode (8), en se fondant sur le report de motif de l'une ou de plusieurs boucles (flottées) manquées (16), boucles repliées (15) boucles unies (14), ou des combinaisons de celles-ci;
- Replacer le fil non-conducteur dans la zone maillée afin qu'il soit lié au fil conducteur;
- Retirer le fil conducteur de plusieurs aiguilles de la zone maillée après avoir garanti la connexion entre les fils conducteur et non-conducteur;
- Couper l'excès de fil, obtenant ainsi une région non-conductrice (4), à l'opposé de l'électrode (8); le fil élastomère reste dans la zone maillée comme indiqué dans les étapes antérieures;
- Appliquer une couche isolatrice comme du silicone en haut de la région conductrice (2) en face de l'électrode maillée et autour de la partie conductrice de l'électrode maillée sur sa partie arrière.

10. Méthode, selon la revendication 9, où les unités répétées de la région conductrice (2) de l'électrode (8) comprennent des structures maillées en trame dans lesquelles les lignes représentent le nombre de courses, les colonnes, le nombre d'aiguilles, un carré noir, une boucle (flottée) manquée (16), le carré noir et blanc, une boucle repliée (15) et le blanc, une boucle unie (14).

11. Méthode, selon les revendications 9 ou 10, où le nombre d'aiguilles lors du maillage simultané est compris entre 5 à 10 afin de garantir la connexion entre les fils conducteur et non-conducteur.
